# DEMANDE DE BREVET EUROPEEN

(11) **EP 4 371 508 A1**
(43) Date de publication de la demande: **22.05.2024**
(21) Numéro de dépôt: 22208155.6
(22) Date de dépôt: 17.11.2022
(51) Int. Cl.: A61B 17/32

(54) **DISPOSITIF DESTINÉ À RÉALISER UNE LIBÉRATION D'UN SYNDROME COMPRESSIF DE LA MAIN**

(71) Demandeur: Keri Medical SA, 1227 Les Acacias (CH)
(72) Inventeur: PRANDI, Bernard, 1227 Les Acacias (CH); MOTTET, Julie, 1227 Les Acacias (CH); APARD, Thomas, 78000 Versailles (FR); COGNET, Jean Michel, 51140 Jonchery sur Vesle (FR)
(74) Mandataire: Micheli & Cie SA

(57) **Abrégé**

La présente invention a pour objet un dispositif destiné à réaliser une libération d'un syndrome compressif de la main comprenant un couteau (2) comprenant un manche (20) et une extrémité de coupe (22) recevant des moyens de coupe (23) et au moins un guide (1) comprenant une portion longitudinale (11) avec une extrémité distale (110) et une extrémité proximale (111) et présentant au moins un canal (113) dans lequel peut coulisser le couteau (2), ledit canal (113) étant délimité par une première et une seconde parois latérales (1133a, 1133b) dont les sommets (1136a, 1136b) définissent un premier plan. Le guide (1) est agencé de sorte que, lors de l'insertion du guide (1) et/ou une fois inséré dans sa position finale dans la zone à traiter de la main, le guide (1) est dans une position dans laquelle le premier plan défini par les sommets (1136a, 1136b) des première et seconde parois latérales (1133a, 1133b) du canal (113 ) forme un angle non nul, de préférence compris entre 4° et 12°, avec le plan défini par la face antérieure de l'avant-bras de la main à traiter.

## Description

La présente invention a pour objet un dispositif destiné à réaliser une libération d'un syndrome compressif de la main, telle la libération du canal carpien.

Le syndrome du canal carpien est un ensemble de signes fonctionnels et physiques liés à la souffrance du nerf médian au niveau du poignet par sa compression et par élévation de la pression au sein du canal carpien. Le canal carpien est la zone de transition entre l'avant-bras et la main. Il est situé dans le talon de la main. Il forme un défilé ostéofibreux inextensible, plus étroit à ce niveau qu'à l'avant-bras ou la main. Le canal carpien est délimité d'une part par les os du carpe qui en constituent le plancher et les côtés, et par le ligament annulaire antérieur du carpe (ou rétinaculum ou ligament rétinaculaire) qui en forme le toit. À l'intérieur de cette structure circulent les tendons fléchisseurs des doigts, le nerf médian et l'artère radiale.

Le syndrome du canal carpien peut résulter de nombreuses maladies, conditions et accidents : œdème, inflammation des tendons, changements hormonaux et mouvements répétés peuvent tous contribuer à l'augmentation de la pression intra canalaire entraînant une compression du nerf médian, responsable du syndrome. Cette compression peut entraîner un engourdissement, des fourmillements dans les doigts, une douleur de la main irradiant au bras et un déficit moteur et peut conduire à la lésion irréversible du nerf.

On dénombre un peu plus de 400 cas pour 100'000 habitants par année aux Etats-Unis, dont un tiers se fait opérer. Le nombre d'opérations pour la France en 2018 était de 125'000. Certains pays reconnaissent le syndrome du canal carpien comme maladie professionnelle.

Quand il y a perte de la sensibilité digitale ou fonte musculaire, le nerf médian est en général sévèrement atteint. Dans les formes légères ou modérées un traitement médical, conservateur, peut être proposé. Dans les formes plus sévères, la chirurgie est préférable. Le traitement chirurgical consiste à sectionner le ligament annulaire antérieur du carpe permettant ainsi la libération du nerf médian des structures qui le compriment. La chirurgie, quelle que soit la technique, à ciel ouvert ou par endoscopie, donne de très bons résultats.

De manière générale, le traitement chirurgical couramment utilisé consiste à réaliser une incision dans le poignet en amont du canal carpien. Une sonde d'échographie ou un endoscope est utilisé pour visualiser le canal et localiser le ligament annulaire antérieur. Un couteau est ensuite inséré dans l'incision pour couper ledit ligament. Certains dispositifs chirurgicaux comprennent un guide inséré avant le couteau et dans lequel ledit couteau peut glisser pour sectionner le ligament. Le guide permet de dilater le canal carpien et de guider le couteau. La coupe du ligament peut se faire de manière antérograde lors de l'insertion du couteau ou de manière rétrograde, le couteau est d'abord inséré dans le canal et la coupe a lieu en sortant le couteau du canal.

Le risque principal lors de ce traitement chirurgical est d'endommager les tissus mous autour du ligament annulaire antérieur, comme le nerf médian ou l'artère radiale lors de l'insertion et du retrait du guide et/ou du couteau.

Le but de la présente invention est de fournir un dispositif destiné à réaliser une libération d'un syndrome compressif de la main, telle la libération du canal carpien, qui permette une protection entière des tissus mous environnants le ligament à sectionner sans complexifier la technique opératoire.

La présente invention a pour objet un dispositif destiné à réaliser une libération d'un syndrome compressif de la main, telle la libération du canal carpien selon la revendication 1.

Les figures annexées illustrent schématiquement deux formes d'exécution d'un dispositif destiné à réaliser une libération d'un syndrome compressif de la main selon l'invention.
La figure 1 illustre le dispositif selon une première forme d'exécution de l'invention.
La figure 2 est un agrandissement de l'extrémité distale du guide du dispositif de la figure 1.
La figure 3 est un agrandissement de l'extrémité de coupe du couteau du dispositif de la figure 1.
Les figures 4a à 7 illustrent les étapes du sectionnement du ligament annulaire antérieur pour la libération du canal carpien.

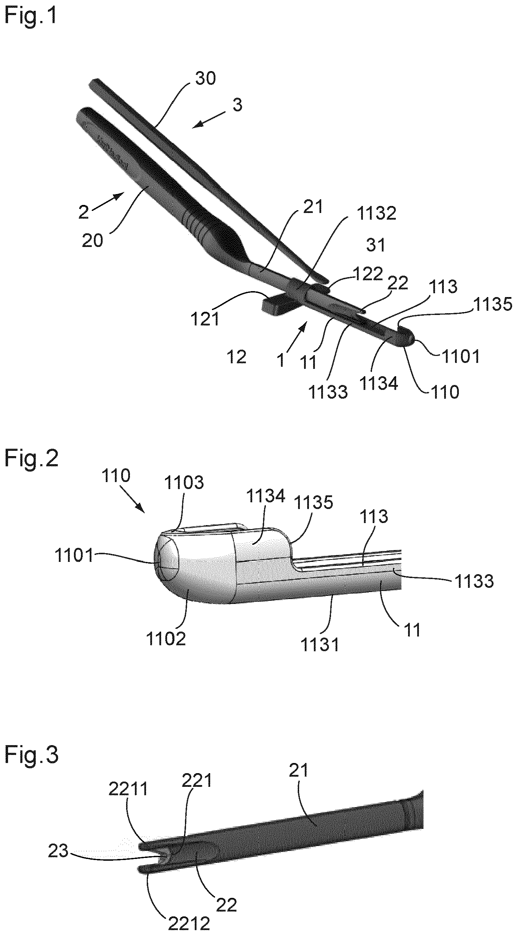

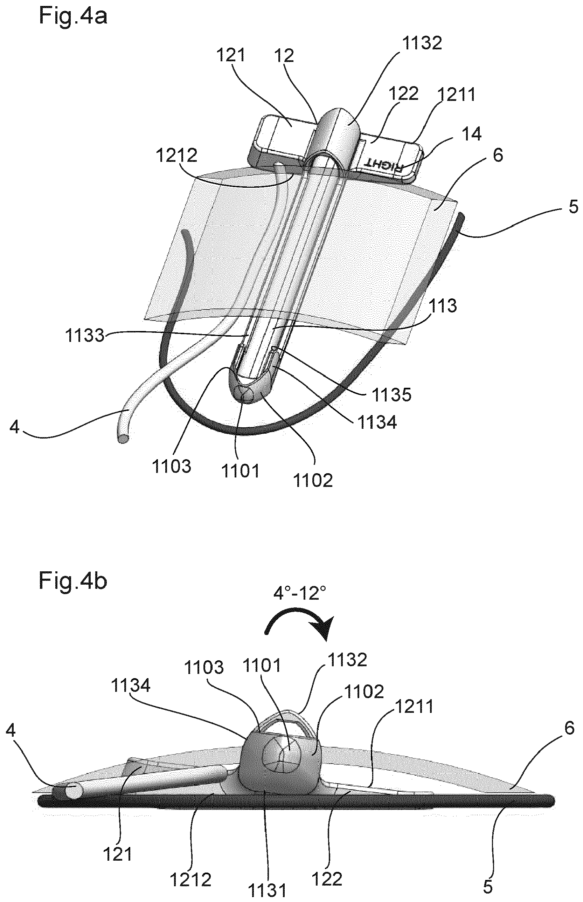

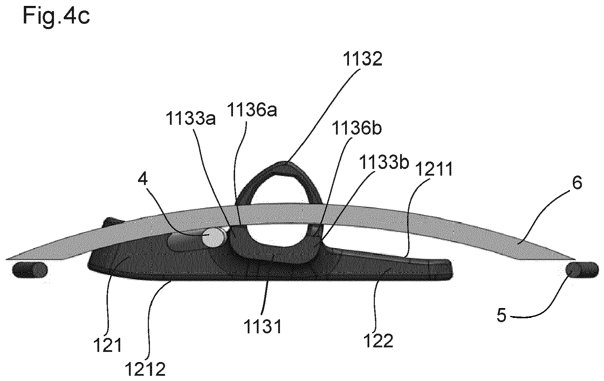

En particulier, les figures 4a et 4b illustrent l'insertion du guide du dispositif de la figure 1 dans le canal carpien vue respectivement en perspective et de face, tandis que la figure 4c est une vue en coupe de la figure 4b selon un axe perpendiculaire à l'axe de la partie longitudinale du guide.

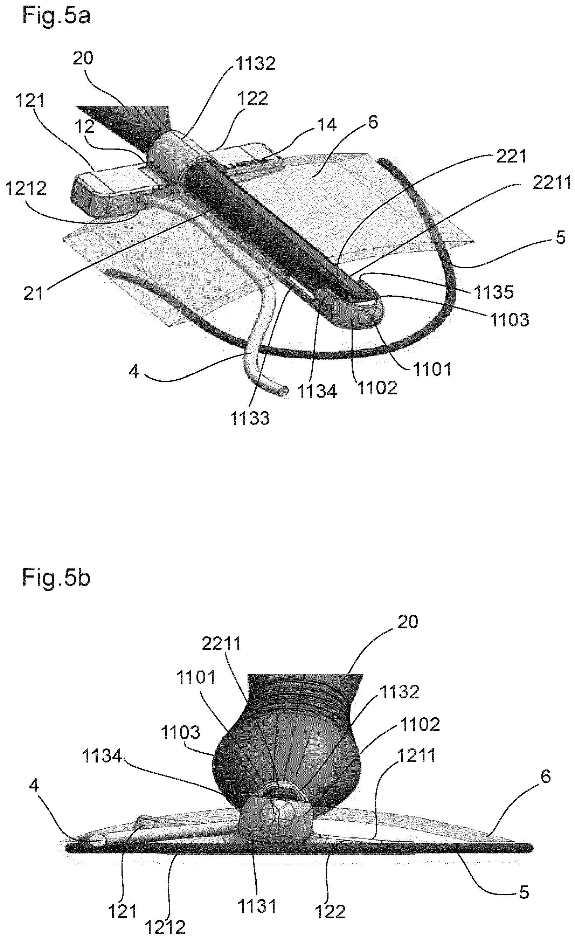

Les figures 5a et 5b illustrent l'insertion du couteau du dispositif de la figure 1 dans le guide pour réaliser la coupe, vue en perspective et de face.

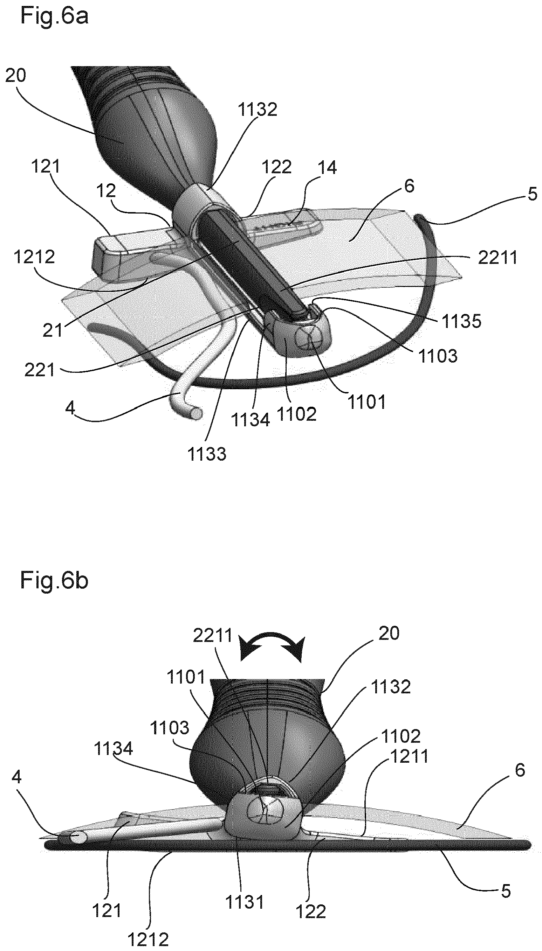

Les figures 6a et 6b illustrent la possibilité pour le praticien d'incliner le couteau dans le guide lors de la coupe, vue respectivement en perspective et de face.

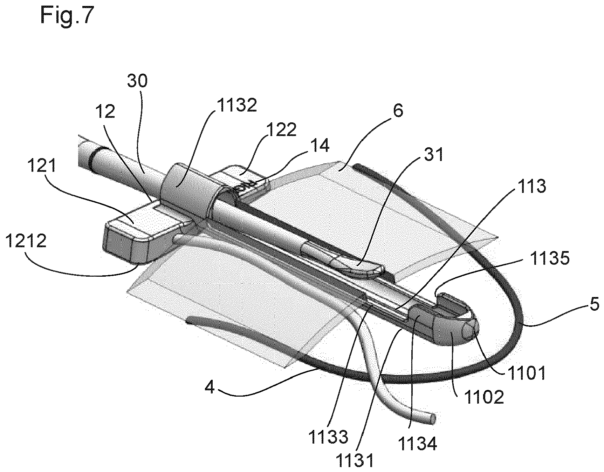

La figure 7 illustre la vérification de la libération complète du ligament par l'insertion de la spatule du dispositif illustré dans la figure 1.

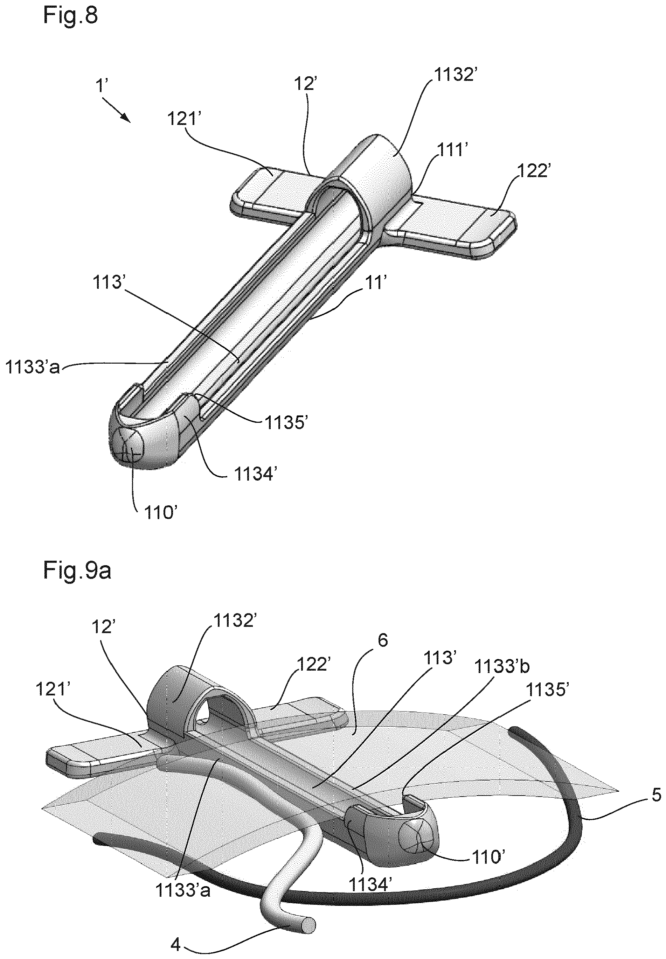

La figure 8 illustre le guide selon une deuxième forme d'exécution de l'invention.

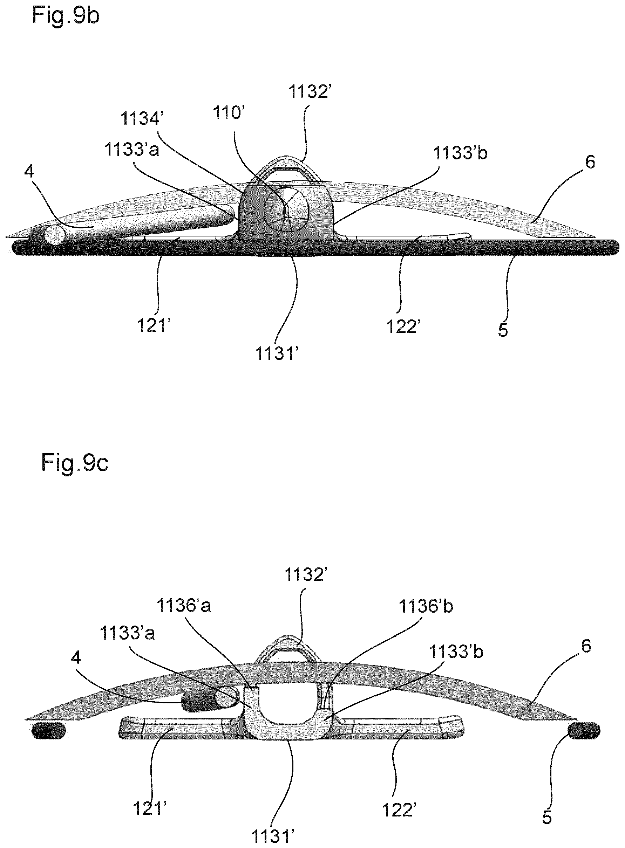

Les figures 9a et 9b illustrent l'insertion du guide de la deuxième forme d'exécution de la figure 8 dans le canal carpien vue respectivement en perspective et de face, tandis que la figure 9c est une vue en coupe de la figure 9b selon un axe perpendiculaire à l'axe de la partie longitudinale du guide.

Dans la première forme d'exécution illustrée aux figures 1 à 7, le dispositif considéré est destiné à réaliser une libération du canal carpien et comprend un couteau 2, un guide 1 et de préférence, une spatule 3.

Le guide 1 comprend une première portion dite longitudinale 11 présentant une extrémité distale 110 et une extrémité proximale. A l'extrémité proximale, le guide 1 comprend une seconde portion transversale 12 formée d'une première et d'une seconde ailettes 121, 122 s'étendant de part et d'autre de la première portion longitudinale 11.

Au moins un canal 113 est formé dans la portion longitudinale 11 du guide 1 et est destiné à recevoir le couteau 2 lors de la procédure de libération du canal carpien. Le canal 113 est fermé vers le bas par son fond 1131 sur toute la longueur de la première portion 11 du guide 1. Le canal 113 est fermé vers le haut et sur les côtés à la hauteur de l'extrémité proximale de la portion longitudinale 11 du guide 1 sur une partie qu'on appellera tunnel d'entrée 1132 du canal 113. Le canal 113 est ouvert vers le haut à la suite du tunnel d'entrée 1132 jusqu'à l'extrémité distale 110.

La hauteur des parois latérales du canal 113 varie sur sa longueur: ledit canal 113 présente des parois latérales hautes 1134 à l'extrémité distale 110 du guide 1 et des parois latérales plus basses 1133a, 1133b entre le tunnel d'entrée 1132 et ladite extrémité distale 110. Le décrochement 1135 formé à la jointure entre les parois latérales hautes 1134 et les parois latérales basses 1133a, 1133b du canal 113 permet de crocheter le ligament 6 devant être sectionner et de le bloquer dans le guide 1 une fois celui-ci en position. Le décrochement 1135 est conformé pour être atraumatique et ne pas endommager les tissus lors du retrait du guide 1.

Dans la première forme d'exécution, les parois latérales basses 1133a, 1133b présentent la même hauteur des deux côtés du canal 113. Ainsi, le plan passant par les sommets 1136a, 1136b des parois latérales basses 1133a, 1133b est parallèle au plan du fond 1131 du canal 113.

En variante, la portion longitudinale 11 du guide 1 pourrait comprendre un second canal destiné à recevoir un instrument comme un endoscope ou un arthroscope et s'étendant parallèlement au premier canal 113. De préférence, les canaux destinés à recevoir le couteau et l'instrument sont alors séparés par une paroi pour éviter toute interférence.

L'extrémité distale 110 de la portion longitudinale 11 du guide 1 présente essentiellement la forme d'une ogive tronquée sur le dessus. En particulier, l'extrémité distale 110, dont un agrandissement est visible sur la figure 2, comprend une pointe 1101 arrondie, légèrement pointue pour faciliter l'insertion du guide, tout en étant atraumatique (pas piquante) pour préserver les tissus lors de l'insertion. La partie 1102 entre la pointe 1101 et le fond 1131 et les parois latérales hautes 1134 du canal 113 est courbée, toujours pour une insertion facile du guide dans le canal carpien. La partie supérieure 1103 de l'extrémité distale 110 est tronquée et plate pour permettre son identification par ultrasons. Cette partie supérieure plate 1103 rejoint les parois latérales hautes 1134 du canal 113. De préférence, la pointe 1101 est à une hauteur minimale par rapport au fond 1131 du canal 113 pour garantir que les tissus ne seront pas endommagés lors de l'insertion du guide 1 dans le canal carpien et en particulier pour éviter que le guide 1 n'attrape l'arche de l'artère radiale 5. Comme on le verra plus loin lors de la description détaillée de l'utilisation du dispositif, l'extrémité distale 110 est également conformée pour servir de butée au couteau 2 lors de la translation de celui-ci dans le canal 113.

La portion transversale 12 et les ailettes 121 et 122 servent à manier le guide pour son insertion et son retrait dans le canal carpien. Selon la première forme d'exécution de l'invention, les ailettes 121, 122 sont agencées de sorte qu'il existe un angle de 4° à 12° entre le plan directeur des ailettes 121, 122 et le plan du fond 1131 du canal 113 ou le plan des sommets 1136a, 1136b des parois latérales basses 1133a, 1133b. Ainsi, lorsque lesdites ailettes 121, 122 reposent sur une surface essentiellement plane comme la face antérieure de l'avant-bras sur laquelle repose les ailettes 121, 122 lors d'un traitement pour la libération du canal carpien, la portion longitudinale 11 et le fond 1131 du canal 113 sont inclinés de 4° à 12° par rapport au plan de la face antérieure de l'avant-bras. Pour ce faire, les ailettes 121, 122 présentent une face supérieure 1211 essentiellement parallèle au fond 1131 du canal 113 et une face inférieure 1212 dont le plan forme un angle compris entre 4° et 12° avec le plan du fond 1131 du canal 113 du la portion longitudinale 11 du guide 1 ou le plan des sommets 1136a, 1136b des parois latérales basses 1133a, 1133b. La face inférieure 1212 est destinée à venir en appui sur la face antérieure de l'avant-bras et de par son angulation déterminer l'inclinaison de la portion longitudinale 11 et du canal 113.

Un autre élément du dispositif selon l'invention est le couteau 2. Comme illustré sur la figure 1, le couteau 2 comprend un manche 20 et une partie active 21. La partie active 21 forme un angle de 20° avec le manche 20. La partie active 21 se termine par une extrémité de coupe 22 qui porte la lame de coupe 23 destinée à sectionner le ligament annulaire antérieur 6. La partie active 21 est destinée à être glissée dans le canal 113 de la portion longitudinale 11 du guide 1.

Dans la forme d'exécution illustrée, le couteau 2 et son extrémité de coupe 22 sont agencés pour réaliser une coupe antérograde, c'est-à-dire lors de l'insertion du couteau dans le canal carpien. En variante, le couteau 2 et son extrémité de coupe 22 pourrait être agencés pour réaliser une coupe rétrograde, c'est-à-dire lors du retrait du couteau du canal carpien.

De préférence, l'extrémité de coupe 22 présente une encoche 221 dans laquelle est retenue la lame de coupe 23. L'encoche 221 forme une languette supérieure 2211 et une languette inférieure 2212 qui protègent la lame de coupe 23 et évitent que celle-ci n'entre en contact avec les tissus environnants le ligament annulaire antérieur 6. De préférence, la lame de coupe 23 est surmoulée dans l'extrémité de coupe 22 dans l'encoche 221.

Enfin, le dispositif selon la forme d'exécution illustrée comprend de préférence une spatule 3. La spatule 3 est un instrument long et effilé comprenant un manche 30 et une extrémité active 31 de forme plate et arrondie. Comme on le verra plus loin, la spatule 3 est destinée dans un premier temps à préparer l'insertion du guide 1 dans le canal carpien et dans un second temps, à vérifier la libération complète du ligament 6. Pour ce faire, la spatule 3 est également adaptée à coulisser dans le canal 113 de la portion longitudinale 11 du guide 1.

Dans la suite, la technique opératoire permettant la libération du canal carpien à l'aide d'un dispositif selon la forme d'exécution de l'invention ci-dessus est décrite en détails en référence aux figures 4a à 7.

Dans l'exemple illustré aux figures, il s'agit de libérer le canal carpien du poignet droit d'un patient.

La première étape consiste à réaliser une incision d'environ 1 cm au pli de flexion du poignet sur le palmaris longus (face antérieure de l'avant-bras). Après avoir exposé et incisé le carpi volare (partie précédant le début du canal carpien), la spatule 3 est insérée sous le ligament annulaire antérieur du carpe 6 pour vérifier qu'il n'y a pas de rameau nerveux transligamentaire (zone de dépression dans le ligament) et pour préparer l'insertion du guide. De préférence, la spatule 3 est ensuite glissée au-dessus du ligament 6 pour faciliter l'avancée ultérieure du couteau 2.

Une sonde échographique est ensuite utilisée pour localiser la zone propice à l'introduction du guide 1 et du couteau 2 sans risquer d'endommager les structures à risque dans et autour du canal carpien comme le nerf médian 4 et l'artère radiale 5.

La guide 1 est ensuite inséré dans cette zone propice sous le ligament annulaire antérieur du carpe 6. Pour ce faire, le praticien empoigne le guide 1 par les ailettes 121, 122 et fait glisser la portion longitudinale 11 du guide 1 dans l'incision. La forme en ogive de l'extrémité distale 110 du guide 1 permet de garantir qu'aucun tissu mou environnant n'est endommagé pendant l'insertion du guide 1. De plus, avec sa pointe 1101 arrondie mais non piquante et haute, ladite extrémité distale 110 ne risque pas d'endommager l'artère radiale 5 qui fait son arche autour du canal carpien. La figure 5b illustre en particulier le guide 1 dans sa position finale dans le canal carpien sous le ligament annulaire antérieur 6. La pointe 1101 passe au-dessus de l'artère radiale 5 et celle-ci a éventuellement pu être repoussée par le guide 1 notamment par la partie 1102 courbée de l'extrémité distale 110.

Le guide 1 est inséré dans le canal carpien jusqu'à sa position finale, c'est-à-dire jusqu'à ce que l'extrémité distale 110 en forme d'ogive passe le ligament 6 et que ledit ligament 6 passe le décrochement 1135 du guide 1, cela correspond en général à une position dans laquelle l'entier de sa portion longitudinale 11 est dans le canal carpien ou encore dans laquelle le tunnel d'entrée 1132 vient en butée contre l'avant-bras. Dans cette position finale, l'extrémité distale 110 est dégagée du ligament 6 qui est crocheté et bloqué dans le canal 113. La figure 4b illustre en particulier la façon dont le décrochement 1135 du canal 113 permet de crocheter le ligament 6 et le bloquer dans le guide 1.

L'insertion du guide 1 dans le canal carpien se fait dans un plan essentiellement parallèle au plan de la face antérieure de l'avant-bras. Selon l'invention, le guide est agencé pour que lors de cette insertion la portion longitudinale 11 et le canal 113 sont inclinés de sorte que le fond 1131 dudit canal n'est pas parallèle au plan de la face antérieure de l'avant-bras mais est incliné de 4° à 12° par rapport audit plan. Cette inclinaison permet de présenter ledit fond 1131 au nerf médian 4 qui va être écarté de la zone de coupe lors de l'insertion du guide, réduisant ainsi les risques de dommages causés à ce nerf 4 lors de la coupe du ligament 6. Les figures 4b et 4c en particulier illustrent le guide 1 selon la première forme d'exécution, en place dans sa position finale (extrémité distale 110 dégagée du ligament 6 et ligament 6 crocheté dans le décrochement 1135). Dans cette position finale, l'inclinaison du canal 113 fait que les parties du guide 1 les plus proches du nerf médian 4 sont les parois latérales basses 1133 et le fond 1131 du canal 113, parties qui empêchent le couteau 2 de venir en contact avec ledit nerf médian 4 lorsque ledit couteau 2 coulisse dans le canal 113 du guide 1.

De manière générale, il est souhaitable que le fond 1131 du canal 113 présente une inclinaison en direction du petit doigt lorsque le guide 1 est inséré dans le canal carpien depuis le poignet en direction des doigts. Ainsi, selon le poignet à traiter, gauche ou droite, il faudra un guide 1 approprié. Le guide 1 peut donc par exemple comprendre une indication 14 sur sa partie transversale 12 permettant d'identifier s'il s'agit d'un guide pour la main gauche ou pour la main droite. Dans la forme d'exécution illustrée, cette indication 14 est le mot « right » gravé sur l'une des ailettes 122.

Une fois le guide 1 inséré dans sa position finale, le praticien peut relâcher les ailettes 121, 122. Dans la première forme d'exécution, de par leur agencement et notamment leur face inférieure 1212, le guide 1 et sa portion longitudinale 11 gardent leur inclinaison de 4° à 12° lorsque ladite face inférieure 1212 vient s'appuyer contre la face antérieure de l'avant-bras.

Comme illustré sur les figures 4a à 4c, lors de l'insertion du guide 1, la portion longitudinale 11 du guide 1, et en particulier, le fond 1131 du canal 113 écartent et protègent le nerf médian 4 qui s'éloigne du canal 113 et de la zone de coupe, réduisant ainsi les risques de dommages causés à ce nerf 4 lors de la coupe du ligament 6.

En variante, le praticien pourrait insérer le guide 1 dans le canal carpien en le faisant glisser sur le fond 1131 du canal 113. Même dans cette situation, grâce à l'agencement de ailettes 121, 122 de la première forme d'exécution, en relâchant le guide 1, la face inférieure 1212 desdites ailettes vient s'appuyer contre la face antérieure de l'avant-bras et fait pivoter le canal 113 de 4° à 12° en direction du petit doigt. Ce pivotement écarte le nerf médian 4 qui fait désormais face au fond 1131 et aux parois latérales 1133, 1134 du canal 113 et est ainsi protégé.

Dans cette position inclinée, le guide 1 est prêt à recevoir le couteau 2 pour la découpe du ligament 6. Avant cela, la sonde d'échographie est à nouveau utilisée pour confirmer que le guide est bien positionné et que la coupe peut avoir lieu sans risque pour les structures environnantes du ligament 6 (nerf médian 4, arcade palmaire). Comme indiqué plus haut, la forme en ogive tronquée de l'extrémité distale 110 du guide 1 et sa partie supérieure 1103 plate permettent de la repérer facilement par échographie.

Le couteau 2 est ensuite introduit dans le guide 1 à travers le tunnel d'entrée 1132 pour coulisser dans le canal 113 et couper le ligament 6 (figures 5a et 5b). Pendant l'insertion du couteau 2, le praticien maintient les ailettes 121, 122 du guide 1. Si le praticien estime qu'il a besoin de plus d'inclinaison du guide, il peut maintenir les ailettes 121, 122 sous contrainte pour obtenir une inclinaison du fond 1131 par rapport au plan de la face antérieure de l'avant-bras supérieure à 12°.

Le couteau 2 est introduit jusqu'à ce son extrémité de coupe 22 vienne en butée dans l'extrémité distale 110 de la portion longitudinale 11 du guide 1, ladite extrémité distale 110 étant conformée pour arrêter toute translation du couteau 2 au-delà de ce point et ainsi protéger l'arche de l'artère radiale 5. En variante, le manche 20 du couteau 2 peut également comprendre une marque indiquant que le couteau 2 ne doit pas être inséré plus avant ou une butée agencée pour coopérer avec le tunnel d'entrée 1132 de l'extrémité proximale 111 de la portion longitudinale 11 du guide 1.

Durant l'insertion du couteau 2, sa partie active 21 et en particulier la languette inférieure 2212 est maintenue en contact avec le fond 1131 du canal 113 du guide 1 pour garantir une coupe sûre. La languette supérieure 2211 du couteau 2 permet de protéger l'aponévrose palmaire superficielle durant la coupe et fermer l'ogive de l'extrémité distale 110 du guide 1 (figure 5a).

De préférence, le couteau 2 et notamment sa partie active 21 et le canal 113 du guide 1 sont agencés pour permettre une rotation dans un ou deux sens de la partie active 21 du couteau 2 dans le canal 113. De préférence, des moyens sont prévus sur la partie active 21 du couteau 2 et/ou dans le canal 113 pour limiter la rotation du couteau 2 par rapport audit canal 113, de préférence à des angles entre 8° et 15°. Ces moyens peuvent consister en un système de forme et contre-forme ou en un système de butée. Cette rotation possible de la partie active 21 du couteau 2 dans le canal 113 a plusieurs avantages. D'une part, elle permet une amorce de la coupe plus aisée. Elle permet d'autre part de débloquer le couteau 2 en cas de coupe plus dure. Ensuite, en pivotant le couteau 2 dans le sens opposé à l'inclinaison du canal 113, l'entraxe entre la languette supérieure 2211 de la partie active 21 du couteau 2 et le fond 1131 du canal 113 est augmenté, ce qui augmente l'épaisseur du ligament 6 admissible dans l'encoche 221 de l'extrémité de coupe 22 du couteau 2. La figure 6b illustre cette contre-rotation du couteau 2 par rapport au canal 113 du guide 1.

Lorsque l'extrémité de coupe 22 du couteau 2 est arrivée en butée dans l'extrémité distale 110 du guide 1, le ligament annulaire antérieur 6 a été coupé. Le couteau 2 peut alors être retiré en maintenant le guide en place, par exemple par ses ailettes 121, 122.

De préférence, pour confirmer la libération complète du ligament 6, la spatule 3 est ensuite insérée dans le guide 1 en la glissant dans le canal 113. L'extrémité distale 110 du guide 1 est conformée pour servir de butée en translation dans le canal 113 à l'extrémité active 31 de la spatule 3 et empêcher ladite extrémité active 31 de causer des dommages à l'artère radiale 5. La spatule 3 est ensuite retirée, avant le retrait du guide 1. La libération du canal carpien est ainsi accomplie en évitant tout dommage aux tissus et structures environnantes sans pour autant complexifier la procédure.

Les figures 8 à 9c illustrent une seconde forme d'exécution du dispositif selon l'invention. Dans cette forme d'exécution, le couteau 2 et la spatule 3 sont identiques à ceux décrits en référence à la première forme d'exécution. Seul le guide change.

De manière similaire à la première forme d'exécution, le guide 1' illustré à la figure 8 comprend une première portion dite longitudinale 11' présentant une extrémité distale 110' et une extrémité proximale. A l'extrémité proximale, le guide 1' comprend une seconde portion transversale 12' formée d'une première et d'une seconde ailettes 121', 122' s'étendant de part et d'autre de la première portion longitudinale 11'.

Au moins un canal 113' est formé dans la portion longitudinale 11' du guide 1' et est destiné à recevoir le couteau 2 lors de la procédure de libération du canal carpien. Le canal 113' est fermé vers le bas par son fond 1131' sur toute la longueur de la première portion 11' du guide 1'. Le canal 113' est fermé vers le haut et sur les côtés à la hauteur de l'extrémité proximale de la portion longitudinale 11' du guide 1' sur une partie qu'on appellera tunnel d'entrée 1132' du canal 113'. Le canal 113' ouvert vers le haut à la suite du tunnel d'entrée 1132' jusqu'à l'extrémité distale 110'.

La hauteur des parois latérales du canal 113' varie sur sa longueur: ledit canal 113' présente des parois latérales hautes 1134' à l'extrémité distale 110' du guide 1' et des parois latérales plus basses 1133' entre le tunnel d'entrée 1132' et ladite extrémité distale 110'. Un décrochement 1135' est formé à la jointure entre les parois latérales hautes 1134' et les parois latérales basses 1133' du canal 113'. Ce décrochement 1135' permet de crocheter le ligament 6 devant être sectionné et de le bloquer dans le guide 1' une fois celui-ci en position. Le décrochement 1135' est conformé pour être atraumatique et ne pas endommager les tissus lors du retrait du guide 1.

Selon la forme d'exécution illustrée aux figures 8 à 9b, les parois latérales basses 1133' ne présentent pas la même hauteur de part et d'autre du canal 113' : une première paroi latérale basse 1133', la paroi basse gauche 1133'a dans les figures, est plus haute que l'autre paroi latérale basse 1133', la paroi basse droite 1133'b. Les parois latérales basses 1133' sont agencées et en particulier présentent une différence de hauteur telle qu'un plan passant par le sommet respectif 1136'a, 1136'b de chacune des dites parois latérales basse 1133a', 1133'b forme un angle compris entre 4° et 12° avec le plan du fond 1131' du canal 113' de la portion longitudinale 11' du guide 1'. Ainsi, lorsque le guide est posé sur une surface plane, son fond 1131' parallèle à ladite surface plane, le plan passant par les sommets 1136'a, 1136'b des parois latérales basse 1133'a et 1133'b forme un angle compris entre 4° et 12° avec ladite surface plane.

Les autres caractéristiques du guide 1' et en particulier son extrémité distale 110' en forme d'ogive sont en tout point similaires à ce qui a été décrit ci-dessus en référence à la première forme d'exécution.

Comme dans la première forme d'exécution, la portion transversale 12' et les ailettes 121' et 122' servent à manier le guide 1' pour son insertion et son retrait dans le canal carpien.

Pour insérer le guide 1' selon cette variante sous le ligament annulaire antérieur du carpe 6, le praticien empoigne le guide 1' par les ailettes 121', 122' et fait glisser la portion longitudinale 11' du guide 1' dans l'incision. La forme en ogive de l'extrémité distale 110' du guide 1' permet de garantir qu'aucun tissu mou environnant n'est endommagé pendant l'insertion du guide 1. Le guide 1' est inséré dans le canal carpien jusqu'à sa position finale, c'est-à-dire jusqu'à ce que l'extrémité distale 110' en forme d'ogive passe le ligament 6 et que ledit ligament 6 passe le décrochement 1135' du guide 1' ; cela correspond en général à une position dans laquelle l'entier de sa portion longitudinale 11' est dans le canal carpien ou encore dans laquelle le tunnel d'entrée 1132' vient en butée contre l'avant-bras. Dans cette position finale, l'extrémité distale 110' est dégagée du ligament 6 qui est crocheté et bloqué dans le canal 113'par le décrochement 1135' comme illustré sur la figure 9a.

L'insertion du guide 1' dans le canal carpien se fait dans un plan essentiellement parallèle au plan de la face antérieure de l'avant-bras. La différence de hauteur entre la paroi basse gauche 1133'a et la paroi basse droite 1133'b, permet de présenter la paroi basse gauche 1133'a plus haute au nerf médian 4 qui va être écarté de la zone de coupe lors de l'insertion du guide, réduisant ainsi les risques de dommages causés à ce nerf 4 lors de la coupe du ligament 6.

Le dispositif destiné à réaliser une libération du canal carpien dans les deux alternatives ci-dessus a été décrit à titre d'exemple uniquement.

En particulier, le dispositif illustré est approprié pour une libération du canal carpien de la main droite. Comme indiqué ci-dessus, un dispositif approprié pour la main gauche est tout à fait similaire, le guide 1 selon la première forme d'exécution étant simplement agencé pour produire une inclinaison du canal 113 dans le sens opposé pour protéger le nerf médian tandis que le guide 1' selon la seconde forme d'exécution présentera soit la paroi basse gauche 1133'a plus basse que la paroi basse droite 1133'b. Le couteau et la spatule ne changent pas. En variante, le dispositif pourrait comprendre un couteau, une spatule et deux guides selon l'une des première ou seconde formes d'exécution, un pour le poignet droite et un pour le poignet gauche.

De préférence, le dispositif est à usage unique et ses composants sont réalisés dans tout matériau approprié à l'usage médical.

Dans les formes d'exécution illustrées, le couteau 2 est agencé pour réaliser une coupe antérograde. En variante, le couteau pourrait être agencé pour réaliser une coupe rétrograde. Dans ce cas, le couteau est introduit dans le canal 113 jusqu'à venir en butée contre l'extrémité distale 110 du guide 1. Avec la forme de ladite extrémité distale et du décrochement 1135, dans cette position de butée l'extrémité de coupe du couteau rétrograde est garantie d'avoir dépassé le ligament 6 qui est crocheté dans le décrochement 1135. Il est alors possible de pivoter le couteau vers le haut du canal 113 pour engager l'extrémité de coupe rétrograde dans le ligament et amorcer la coupe. La rotation du couteau par rapport au canal 113 selon l'axe longitudinal dudit couteau présente les même avantages que dans le cas d'une découpe antérograde : amorce de coupe facilitée, déblocage possible en cas de coupe dure, possible d'admettre des ligaments plus épais en pivotant le couteau dans le sens opposé à l'inclinaison du guide. Il en va de même avec un guide selon la seconde forme d'exécution de l'invention.

En variante, la portion longitudinale du guide pourrait comprendre un second canal distinct du premier, séparé du premier par une cloison et destiné à recevoir un instrument comme une caméra endoscopique.

En variante, le dispositif pourrait ne pas comprendre de spatule.

En variante, le dispositif pourrait ne pas comprendre de décrochement 1135.

De manière générale, le dispositif destiné à réaliser une libération d'un syndrome compressif de la main comprend :
- Un couteau comprenant un manche et une extrémité de coupe recevant des moyens de coupe ; et
- Un guide comprenant une portion longitudinale avec une extrémité distale et une extrémité proximale et présentant au moins un canal dans lequel peut coulisser le couteau, ledit canal étant délimité par une première et une seconde parois latérales dont les sommets définissent un premier plan.

Selon l'invention, le guide est agencé de sorte que, lors de l'insertion du guide et/ou une fois inséré dans sa position finale dans la zone à traiter de la main, le guide est dans une position dans laquelle le premier plan passant par les sommets des première et seconde parois latérales du canal forme un angle non nul, de préférence compris entre 4° et 12°, avec le plan défini par la face antérieure de l'avant-bras de la main à traiter.

Ainsi, dans la position finale du guide et/ou lors de son insertion, le premier plan passant par les sommets des première et second parois latérales du canal est incliné dans le canal carpien ce qui permet d'écarter le nerf médian dudit canal lors de l'insertion du guide et de protéger ledit nerf médian en lui présentant le fond du canal et/ou une des première ou second paroi latérale.

Selon une première alternative de l'invention, le guide comprend en outre une portion transversale s'étendant de part et d'autre de l'extrémité proximale. La portion transversale est destinée à venir en appui sur une face essentiellement plane du bras ou de la main et est agencée de sorte que lorsque la portion transversale est en appui sur un second plan, il existe un angle non nul, de préférence compris entre 4° et 12°, entre ledit second plan et le premier plan. Dans cette alternative, le premier plan est parallèle au plan du fond du canal.

Ainsi, dans cette première alternative, lorsque la portion transversale du guide vient en appui sur la face antérieure de l'avant-bras, le canal est incliné dans le canal carpien ce qui permet d'écarter le nerf médian dudit canal lors de l'insertion du guide et de protéger ledit nerf médian en lui présentant le fond du canal.

Selon une seconde alternative, les première et seconde parois latérales sont agencées de sorte que la première desdites parois latérales présente, sur la majeure partie de la longueur de la portion longitudinale du guide, une hauteur supérieure à la seconde paroi latérale. La différence de hauteur entre les première et seconde parois latérales est telle que le premier plan passant par le sommet de chacune desdites parois latérales forme un angle compris entre 4° et 12° avec le plan du fond du canal de la portion longitudinale du guide. En d'autres termes, cette différence de hauteur permet au le premier plan défini par les sommets des première et second parois latérale de former un angle non nul, de préférence compris entre 4° et 12°, avec le plan défini par la face antérieure de l'avant-bras de la main à traiter, lors de l'insertion dudit guide dans la zone à traiter.

Ainsi, dans cette seconde alternative également, lors de l'insertion du guide et/ou dans sa position finale, la différence de hauteur entre les première et seconde parois latérales du canal la première paroi latérale et l'inclinaison du premier plan défini par le sommet desdites parois latérales permet d'écarter le nerf médian dudit canal et de protéger ledit nerf médian en lui présentant la première paroi latérale la plus haute.

Le dispositif selon l'invention peut comprendre en outre les caractéristiques optionnelles suivantes quelle que soit l'alternative utilisée pour le guide.

De préférence, l'extrémité distale de la portion longitudinale du guide présente essentiellement une forme d'ogive avec une pointe arrondie et une partie courbée entre le fond, les parois latérales du canal et ladite pointe. De préférence, la distance entre la pointe et le fond du canal est la plus grande possible. En outre, ladite extrémité distale est agencée pour empêcher la translation du couteau et de son extrémité de coupe hors du canal. Ainsi, l'extrémité distale est conformée pour préserver les tissus lors de l'insertion du guide dans la zone de coupe et lors de l'insertion du couteau dans le guide en servant de butée à ce dernier.

De préférence, l'extrémité distale a une forme d'ogive tronquée avec une partie supérieure plate pour permettre son identification par ultrasons.

De préférence, le couteau comprend une languette inférieure destinée à coulisser contre le fond du canal et une languette supérieure destinée à faciliter l'insertion du couteau et à protéger les tissus environnants la zone de coupe, une encoche entre lesdites languettes reçoit une lame de coupe et forme l'extrémité de coupe du couteau. La languette supérieure est agencée pour venir fermer l'extrémité distale du guide lorsque l'extrémité de coupe du couteau est en butée dans ladite extrémité distale. Les tissus environnants sont donc protégés à 360° des moyens de coupe.

De préférence, le couteau et le guide sont agencés pour que ledit couteau puisse pivoter dans le canal de la portion longitudinale du guide dans un sens ou dans l'autre, d'un angle compris de préférence entre 8° et 15°. Cette rotation permet de faciliter l'amorce de coupe et débloquer le couteau en cas de coupe dur. De plus, selon le sens de rotation du couteau par rapport au sens d'inclinaison du guide selon la première alternative, il est possible d'augmenter l'épaisseur du ligament recevable dans l'extrémité de coupe.

De préférence, le dispositif comprend en outre une spatule comportant un manche et une extrémité active plate et arrondie. La spatule est agencée pour pouvoir coulisser dans le canal du guide jusqu'à venir en butée contre l'extrémité distale de la portion longitudinale du guide, l'empêchant ainsi de sortir du canal et protégeant les tissus environnant de la zone de coupe. La spatule utilisée seule permet de préparer la zone de coupe pour l'insertion du guide et le retrait du couteau et, utilisée avec le guide, permet de vérifier la libération complète du ligament compressif.

De préférence, il existe un décrochement entre l'extrémité distale de la portion longitudinale du guide et le reste du canal, les parois latérales dudit canal étant plus basses que les parois latérales de l'extrémité distale. Ledit décrochement permet de crocheter le ligament à couper pour le bloquer dans le guide lorsque celui-ci est inséré sous ledit ligament.

On réalise ainsi un dispositif destiné à réaliser une libération d'un syndrome compressif de la main et notamment une libération du canal carpien, qui permet une protection entière des tissus mous environnants la zone de coupe et qui garantit une coupe sûre et efficace sans complexifier la technique opératoire.

## Revendications

1. Dispositif destiné à réaliser une libération d'un syndrome compressif de la main comprenant :
• un couteau (2) comprenant un manche (20) et une extrémité de coupe (22) recevant des moyens de coupe (23) ; et
• un guide (1 ;1') comprenant une portion longitudinale (11 ;11') avec une extrémité distale (110 ; 110') et une extrémité proximale (111 ; 111') et présentant au moins un canal (113 ; 113') dans lequel peut coulisser le couteau (2), ledit canal (113 ; 113') étant délimité par une première et une seconde parois latérales (1133a, 1133b; 1133'a, 1133'b) dont les sommets (1136a, 1136b; 1136'a, 1136'b) définissent un premier plan ,
**caractérisé par le fait que** le guide (1 ; 1') est agencé de sorte que, lors de l'insertion du guide (1 ; 1') et/ou une fois inséré dans sa position finale dans la zone à traiter de la main, le guide (1 ; 1') est dans une position dans laquelle le premier plan défini par les sommets (1136a, 1136b ; 1136'a, 1136'b) des première et seconde parois latérales (1133a, 1133b ; 1133'a, 1133'b) du canal (113 ; 113') forme un angle non nul, de préférence compris entre 4° et 12°, avec le plan défini par la face antérieure de l'avant-bras de la main à traiter.

2. Dispositif selon la revendication 1, **caractérisé par le fait que** le guide (1) comprend en outre une portion transversale (12) s'étendant de part et d'autre de l'extrémité proximale (11) ; et **par le fait que** la portion transversale (12) est destinée à venir en appui sur une face essentiellement plane du bras ou de la main et est agencée de sorte que lorsque la portion transversale est en appui sur un second plan, il existe un angle non nul, de préférence compris entre 4° et 12°, entre ledit second plan et le premier plan défini par les sommets (1136a, 1136b) des première et seconde parois latérales (1133a, 1133b ; 1133'a, 1133'b), le premier plan étant parallèle au plan défini par le fond (1131) du canal (113).

3. Dispositif selon la revendication 2, **caractérisé par le fait que** la portion transversale (11) comprend des ailettes (121, 122) présentant une face supérieure (1211) essentiellement parallèle au premier plan et une face inférieure (1212) formant un angle compris entre 4° et 12° avec le premier plan, la face inférieure (1212) étant celle destinée à venir en appui sur la face antérieure de l'avant-bras.

4. Dispositif selon la revendication 1, **caractérisé par le fait que** les première et une seconde parois latérales (1133'a, 1133'b) sont agencées de sorte que la première (1133'a) desdites parois latérales présente, sur la majeure partie de la longueur de la portion longitudinale (11') du guide (1'), une hauteur supérieure à la seconde paroi latérale (1133b') ; et de sorte que la différence de hauteur entre les première et seconde parois latérales (1133'a, 1133'b) est telle que le premier plan passant par le sommet de chacune desdites parois latérales (1133'a, 1133'b) forme un angle compris entre 4° et 12° avec le plan du fond (1131') du canal (113') et/ou avec le plan défini par la face antérieure de l'avant-bras de la main à traiter, lors de l'insertion du guide (1') et/ou une fois ledit guide (1') inséré dans sa position finale dans la zone à traiter de la main.

5. Dispositif selon l'une des revendications précédentes, **caractérisé par le fait que** l'extrémité distale (110 ; 110') de la portion longitudinale (11 ; 11') du guide (1 ; 1') présente essentiellement une forme d'ogive avec une pointe arrondie (1101) atraumatique et une partie courbée (1102) entre le fond (1131 ; 1131'), les première et seconde parois latérales (1133, 1134 ; 1133', 1134') du canal (113 ; 113') et ladite pointe (1101).

6. Dispositif selon la revendication 5, **caractérisé par le fait que** la distance entre la pointe (1101) et le fond (1131 ; 1131') du canal (113 ; 113') est la plus grande possible.

7. Dispositif selon l'une des revendications précédentes, **caractérisé par le fait que** ladite extrémité distale (110 ; 110') est agencée pour empêcher la translation du couteau (2) et de son extrémité de coupe (22) hors du canal (113 ; 113').

8. Dispositif selon l'une des revendications précédentes, **caractérisé par le fait que** l'extrémité distale (110 ; 110') a une forme d'ogive tronquée avec une partie supérieure plate (1103) pour permettre son identification par ultrasons.

9. Dispositif selon l'une des revendications précédentes, **caractérisé par le fait que** le couteau (2) comprend une languette inférieure (2212) destinée à coulisser contre le fond (1131 ; 1131') du canal (113 ; 113') et une languette supérieure (2211) destinée à faciliter l'insertion du couteau et à protéger les tissus environnants la zone de coupe, une encoche (221) entre lesdites languettes reçoit une lame de coupe (23) et forme l'extrémité de coupe (22) du couteau.

10. Dispositif selon l'une des revendications précédentes, **caractérisé par le fait que** la languette supérieure (2211) est agencée pour venir fermer l'extrémité distale (110 ; 110') du guide lorsque l'extrémité de coupe du couteau est en butée dans ladite extrémité distale.

11. Dispositif selon l'une des revendications précédentes, **caractérisé par le fait que** le couteau (2) et le guide (1 ; 1') sont agencés pour que ledit couteau (2) puisse pivoter dans le canal (113 ; 113') de la portion longitudinale (11 ; 11') du guide (1 ; 1') dans un sens ou dans l'autre, d'un angle compris de préférence entre 8° et 15°.

12. Dispositif selon l'une des revendications précédentes, **caractérisé par le fait qu'**il comprend en outre une spatule (3) comportant un manche (30) et une extrémité active (31) plate et arrondie ; et **par le fait que** la spatule (3) est agencée pour pouvoir coulisser dans le canal (113 ; 113') du guide jusqu'à venir en butée contre l'extrémité distale (110 ; 110') de la portion longitudinale (11 ; 11') du guide (1 ; 1').

13. Dispositif selon l'une des revendications précédentes, **caractérisé par le fait que** le canal (113 ; 113') présente à son extrémité distale des parois latérales hautes (1134 ; 1134') dont la hauteur est plus élevées que celles des première et seconde parois latérales (1133 ; 1133'), de sorte qu'il existe un décrochement (1135 ; 1135') entre l'extrémité distale (110 ; 110') de la portion longitudinale (11 ; 11') du guide (1 ; 1') et le reste du canal (113 ; 113'), ledit décrochement (1135 ; 1135') permettant de crocheter le ligament (6) à couper pour le bloquer dans le guide (1 ; 1') lorsque celui-ci est inséré sous ledit ligament.
